# EUROPEAN PATENT SPECIFICATION

(11) **EP 3 190 216 B1**
(45) Date of publication and mention of the grant of the patent: **08.05.2019**
(21) Application number: 16842358.0
(22) Date of filing: 02.09.2016
(51) Int. Cl.: D04H 1/4291, D04H 1/724, D01D 5/18, D04H 13/00, A61F 13/53, D04H 1/4326, D01F 6/16, D01F 6/36

(54) **METHOD FOR MANUFACTURING SUPER ABSORBENT POLYMER FIBER**
VERFAHREN ZUR HERSTELLUNG EINER SUPERSAUGFÄHIGEN POLYMERFASER
PROCÉDÉ DE FABRICATION D'UNE FIBRE EN POLYMÈRE SUPERABSORBANT

(30) Priority: 04.09.2015 KR 20150125535; 22.08.2016 KR 20160106096
(43) Date of publication of application: 12.07.2017
(73) Proprietor: LG Chem, Ltd., Seoul 07336 (KR)
(72) Inventor: SEO, Jin-Seok, Daejeon 34122 (KR); KIM, Young Sam, Daejeon 34122 (KR)
(74) Representative: Goddar, Heinz J.
(86) International application number: PCT/KR2016/009856
(87) International publication number: WO 2017/039392

(56) References cited:
- WO-A1-2013/083698
- CN-B- 103 160 952
- CN-B- 103 160 952
- JP-A- 2002 540 302
- KR-A- 20080 030 939
- US-A1- 2004 265 612
- US-A1- 2008 081 189

## Description

### Technical Field

The present invention relates to a method of manufacturing a superabsorbent polymer fiber.

### Background Art

Superabsorbent polymers (SAPs) are synthetic polymer materials that are able to absorb about 500 to 1000 times their own weight in moisture. Such superabsorbent polymers have begun to be used in real-world applications for sanitary items, and are currently being widely employed not only in hygiene products, such as disposable baby diapers and the like, but also in soil conditioners for gardening applications, water-stopping agents for civil engineering and construction applications, sheets for raising seedlings, freshness preservatives for food distribution, fomentation materials, etc. Accordingly, superabsorbent polymers (SAPs), which are known to have outstanding absorption capability compared to existing absorbents, are utilized in a wide variety of applications, and thus the market competitiveness thereof is regarded as high.

Currently useful superabsorbent polymers are prepared in the form of a powder. Such a superabsorbent polymer powder may be scattered or may leak when manufacturing hygiene materials or upon real-world application, and the use range thereof is limited because it has to be used together with a specific type of substrate. Furthermore, the process of manufacturing a superabsorbent polymer powder is complicated and involves many factors that must be controlled.

CN103160952B relates to a preparation method for high water-absorption fiber, wherein the high water-absorption fiber is prepared by a rear cross linking agent. US2008081189A1 relates to a method for making mixed polymer composite fibers, comprising blending a carboxyalkyl cellulose and a galactomannan polymer or glucomannans polymer in water to provide an aqueous solution; treating the aqueous solution with a first crosslinking agent to provide a gel; forming gel fibers from the get using melt blowing, centrifugal spinning, wet spinning or dry-jet wet spinning; treating the gel fibers with a water miscible solvent to provide mixed polymer composite fibers.

### Disclosure

### Technical Problem

Therefore, the present invention has been made keeping in mind the problems encountered in the related art, and the present invention is intended to provide a novel type of fiber having a superabsorbent polymer function, which entails no concern of scattering or leaking because it is provided in the form of a nonwoven fabric, unlike a superabsorbent polymer powder, and moreover which may be directly spun on a substrate during the production thereof, thus simplifying the manufacturing process. In addition, the present invention is intended to provide a method of manufacturing a superabsorbent polymer fiber, the application range of which is broad thanks to the flexibility thereof.

### Technical Solution

Therefore, the present invention provides a method of manufacturing a superabsorbent polymer fiber, comprising the steps of: (1) preparing a neutralization solution by dissolving a polymer of a water-soluble ethylenic unsaturated monomer in a sodium hydroxide aqueous solution; (2) preparing a spinning solution by adding the neutralization solution with a crosslinking agent and performing stirring; and (3) producing a superabsorbent polymer fiber by subjecting the spinning solution to centrifugal spinning using a spinneret and then performing drying, wherein the neutralization solution has a neutralization degree of 40 to 90 mol%, wherein the water-soluble ethylenic unsaturated monomer is at least one selected from the group consisting of isobutylene, maleic anhydride, acrylic acid, methylacrylate, and hydroxypropyl methacrylate, and wherein the crosslinking agent is an epoxy compound.

In addition, the present invention provides a superabsorbent polymer fiber, manufactured by the aforementioned method.

### Advantageous Effects

According to the present invention, a method of manufacturing a superabsorbent polymer fiber is advantageous in that a centrifugal spinning process is performed, and thus the device configuration is simple, energy consumption is low, fewer limitations are imposed on the spinning polymer, and the manufacturing process is simple due to the formation of a nonwoven fabric. Furthermore, unlike a superabsorbent polymer in powder form, the novel type of fiber having a superabsorbent polymer function can be provided in the form of a nonwoven fabric, thus causing no concern of scattering or leaking upon production thereof. Also, in the present invention, direct spinning on a substrate is possible during the production thereof, thus simplifying the manufacturing process, and the superabsorbent polymer fiber, which is flexible and has a variety of applications, can be obtained, thus enabling the expansion thereof to new application fields.

### Brief Description of Drawings

FIG. 1 shows a photograph of a superabsorbent polymer fiber (nonwoven fabric) according to an embodiment of the present invention; and
FIG. 2 shows a scanning electron microscope image of a superabsorbent polymer fiber (nonwoven fabric) according to an embodiment of the present invention.

### Best Mode

Hereinafter, a detailed description will be given of the present invention.

The present invention addresses a method of manufacturing a superabsorbent polymer fiber, comprising the steps of: (1) preparing a neutralization solution by dissolving a polymer of a water-soluble ethylenic unsaturated monomer in a sodium hydroxide aqueous solution; (2) preparing a spinning solution by adding the neutralization solution with a crosslinking agent and performing stirring; and (3) producing a superabsorbent polymer fiber by subjecting the spinning solution to centrifugal spinning using a spinneret and then performing drying, wherein the neutralization solution has a neutralization degree of 40 to 90 mol%, wherein the water-soluble ethylenic unsaturated monomer is at least one selected from the group consisting of isobutylene, maleic anhydride, acrylic acid, methylacrylate, and hydroxypropyl methacrylate, and wherein the crosslinking agent is an epoxy compound.

Specifically, in step (1) of the method of manufacturing the superabsorbent polymer fiber according to the present invention, the neutralization solution is prepared by dissolving a polymer of a water-soluble ethylenic unsaturated monomer in a sodium hydroxide aqueous solution.

In the method of manufacturing the superabsorbent polymer fiber according to the present invention, the water-soluble ethylenic unsaturated monomer is at least one selected from the group consisting of isobutylene, maleic anhydride, acrylic acid, methyl acrylate, and hydroxypropyl methacrylate. Acrylic acid is most preferable. When acrylic acid is used as the monomer, a superabsorbent polymer fiber having improved absorption capability may be obtained. Meanwhile, in the method of manufacturing the superabsorbent polymer fiber according to the present invention, the concentration of the water-soluble ethylenic unsaturated monomer may be appropriately determined taking into consideration the reaction time and the reaction conditions, and the amount of the water-soluble ethylenic unsaturated monomer is preferably set to the range of 10 to 50 wt% based on the total weight of the sodium hydroxide aqueous solution. If the concentration of the water-soluble ethylenic unsaturated monomer is less than 10 wt%, economic benefits may be negated. On the other hand, if the concentration thereof exceeds 50 wt%, viscosity may increase and thus the spinning solution may not be spun via the spinneret, making it impossible to form a fiber phase.

The neutralization degree of the neutralization solution prepared in step (1) is set within the range of 40 to 90 mol%, and more preferably 50 to 80 mol%. As used herein, the term "neutralization degree" is a value calculated using a predetermined equation upon the measurement of a water-soluble component. As the neutralization degree decreases, the absorption capability of the ultimately obtained superabsorbent polymer fiber may decrease.

In step (2) of the present invention, the spinning solution is prepared by adding the neutralization solution obtained in step (1) with the crosslinking agent and stirring it.

The crosslinking agent, which is added in the present invention is an epoxy compound.

Also, examples of the epoxy compound may include ethylene glycol diglycidyl ether and glycidol, and the polyamine compound may include at least one selected from the group consisting of ethylene diamine, diethylene triamine, triethylene tetramine, tetraethylene pentamine, pentaethylene hexamine, polyethyleneimine, and polyamide polyamine.

The amount of the crosslinking agent that is added to treat the surface of the polymer particles may be appropriately determined depending on the kind of crosslinking agent or the reaction conditions, and is generally set to the range of 0.001 to 5 wt%, preferably 0.01 to 3 wt%, and more preferably 0.05 to 2 wt%, based on the total weight of the water-soluble ethylenic unsaturated monomer. If the amount of the crosslinking agent is too small, the crosslinking reaction does not readily occur. On the other hand, if the amount thereof exceeds 5 wt% based on the total weight of the water-soluble ethylenic unsaturated monomer, the properties of the superabsorbent polymer may deteriorate somewhat due to the excessive crosslinking reaction.

In the following step (3), the spinning solution obtained in step (2) is placed in a spinneret so as to be centrifugally spun and is then dried, thereby producing a superabsorbent polymer fiber.

The centrifugal spinning is a process of producing a nonwoven fabric in a manner in which a polymer that is melted or dissolved is placed in a spinneret having multiple holes and rotated at high speed and the polymer that is not solidified is stretched using centrifugal force acting upon rotation, whereby refined and solidified fibers are stacked on a collector. Centrifugal spinning is advantageous in that the device configuration is simple, energy consumption is low, there are few limitations on the polymers that are usable, and the manufacturing process is simple due to the formation of a nonwoven fabric.

In an embodiment of the present invention, the rotation speed during the centrifugal spinning process is preferably set to, but is not limited to, the range of 3,000 rpm to 15,000 rpm. Upon centrifugal spinning at the above rotation speed, direct spinning on a substrate is possible, thus simplifying the manufacturing process.

In step (3), the drying may be performed at a drying temperature of 100 to 250°C for 10 min to 120 min.

As used herein, the term "drying temperature" refers to the temperature of a heat medium supplied for the drying process or the temperature of a drying reactor containing a heat medium and a polymer in the drying process. If the drying temperature is lower than 100°C, the drying time may become excessively long, and the properties of the ultimately obtained superabsorbent polymer fiber may be deteriorated. On the other hand, if the drying temperature is higher than 250°C, only the surface of the fiber may be excessively dried, and thereby the properties of the ultimately obtained superabsorbent polymer fiber may be deteriorated. The drying is preferably carried out at a temperature of 100 to 250°C, and more preferably 160 to 200°C.

The drying time is not limited, but is preferably set to the range of 10 min to 120 min, and more preferably 20 min to 90 min, taking processing efficiency into account. Also, the drying process is not limited, so long as it is typically used in the art. Specifically, the drying process may be performed using hot air supply, IR irradiation, microwave irradiation, or UV irradiation.

Provided is a fiber that is preferably a nonwoven fabric, but is not limited thereto.

Provided is a superabsorbent polymer fiber that is suitable for use in hygiene materials or resin-molded products. Here, the resin-molded products may include the polymer fiber of the aforementioned embodiment, or may be composed exclusively of such a polymer fiber.

The end use of such a disposable resin-molded product is not particularly limited, but may encompass molded products useful in various fields, such as medical, chemical, chemical engineering, food or cosmetic fields.

In addition, the present invention addresses a superabsorbent polymer fiber manufactured by the above method.

### Mode for Invention

A better understanding of the present invention may be obtained via the following examples, which are set forth to illustrate, but are not to be construed as limiting the scope of the present invention. The scope of the present invention is given by the claims, and also contains all modifications within the meaning and range equivalent to the claims. Unless otherwise mentioned, "%" and "part", indicating amounts in the following examples and comparative examples, are given on a mass basis.

### Examples

### Example 1. Production of superabsorbent polymer fiber

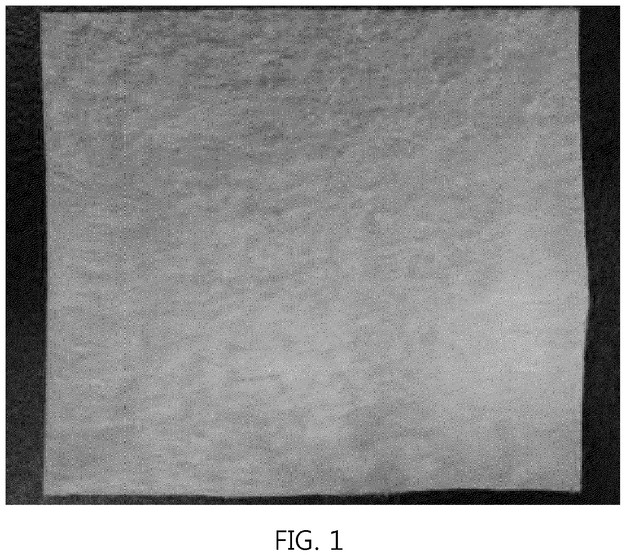

100 g of isobutylene/maleic anhydride and 41.5 g of caustic soda (sodium hydroxide) were dissolved in 330 g of water to give a solution having a solid content of 30 wt% and a neutralization degree of 80 mol%. This solution was then added with, as a crosslinking agent, epoxy (ethylene glycol diglycidyl ether, EX-810) in an amount of 0.3 wt%, based on the amount of isobutylene/maleic anhydride, and stirred so as to be completely mixed, thus preparing a spinning solution. 5 g of the spinning solution was placed in a spinneret having a hole size of 600 µm and spun at 10,000 rpm, whereby a sample was collected and then dried at 190°C for 30 min, thereby manufacturing a superabsorbent polymer fiber nonwoven fabric. The nonwoven fabric thus obtained is shown in FIG. 1.

### Example 2. Production of superabsorbent polymer fiber

A superabsorbent polymer fiber nonwoven fabric was manufactured in the same manner as in Example 1, with the exception that the epoxy serving as the crosslinking agent was added in an amount of 1 wt% based on the amount of isobutylene/maleic anhydride.

### Example 3. Production of superabsorbent polymer fiber

100 g of a copolymer comprising acrylic acid, methyl acrylate, and hydroxypropyl methacrylate and 27.8 g of caustic soda were dissolved in 383.25 g of water to give a solution having a solid content of 25 wt% and a neutralization degree of 50 mol%. This solution was then added with, as a crosslinking agent, epoxy (ethylene glycol diglycidyl ether, EX-810) in an amount of 0.1 wt% based on the amount of the copolymer, and stirred so as to be completely mixed, thus preparing a spinning solution. 5 g of the spinning solution was placed in a spinneret having a hole size of 600 µm and spun at 7,000 rpm, whereby a sample was collected and then dried at 140°C for 30 min, thereby manufacturing a superabsorbent polymer fiber nonwoven fabric.

### Comparative Example 1. Production of superabsorbent polymer fiber

A superabsorbent polymer fiber nonwoven fabric was manufactured in the same manner as in Example 2, with the exception that a solution having a neutralization degree of 35 mol% was spun.

### Comparative Example 2. Production of superabsorbent polymer fiber

A superabsorbent polymer fiber nonwoven fabric was manufactured in the same manner as in Example 2, with the exception that a solution having a neutralization degree of 95 mol% was spun.

The conditions of Examples 1 to 4 and Comparative Example 1 and 2 are shown in Table 1 below.

**[Table 1]**

| | A concentration (wt%) | B concentration (wt%) | Neutralization degree (mol%) | Rpm | Drying temperature (°C) | Drying time (min) |
|---|---|---|---|---|---|---|
| Ex.1 | 30 | 0.3 | 80 | 10,000 | 190 | 30 |
| Ex.2 | | 1 | | | | |
| | | | | | | |
| Ex.3 | 25 | 0.1 | 50 | 7,000 | 140 | |
| C.Ex.1 | 30 | 1 | 35 | 10,000 | 190 | |
| C.Ex.2 | 30 | 1 | 95 | 10,000 | 190 | |

| | | | | | | |
|---|---|---|---|---|---|---|
| A: Water-soluble ethylenic unsaturated monomer B: Crosslinking agent | | | | | | |

### Test Examples

### Test Example 1. Evaluation of properties of superabsorbent polymer fiber nonwoven fabric - Centrifugal Retention Capacity (CRC)

The superabsorbent polymer fiber nonwoven fabrics of Examples 1 to 4 were measured for CRC. CRC was measured using the EDANA method WSP 241.3. Specifically, 0.2 g of the prepared superabsorbent nonwoven fabric was placed in a teabag and then immersed in a 0.9% saline solution for 30 min. Thereafter, dehydration was performed for 3 min at a centrifugal force of 250 G (gravity), and the amount of saline solution that was absorbed was measured.

### Test Example 2. Evaluation of properties of superabsorbent polymer fiber nonwoven fabric - Absorption Under Load (AUL)

The superabsorbent nonwoven fabrics of Examples 1 to 4 were measured for AUL. AUL was measured using the EDANA method WSP 242.3. Specifically, 0.16 g of a sample of the prepared superabsorbent nonwoven fabric was placed in a cylinder according to EDANA, and a pressure of 0.3 psi was applied using a piston and a weight. Thereafter, the amount of 0.9% saline that was absorbed in 60 min was measured.

**[Table 2]**

| | CRC (g/g) | AUL (g/g) |
|---|---|---|
| Ex.1 | 61.6 | 7.6 |
| Ex.2 | 24.4 | 22.2 |
| | | |
| Ex.3 | 19.7 | 19.2 |
| C.Ex.1 | 16.3 | 16.7 |
| C.Ex.2 | 22.5 | 20.4 |

### Test Example 3. Scanning Electron Microscope (SEM) Analysis

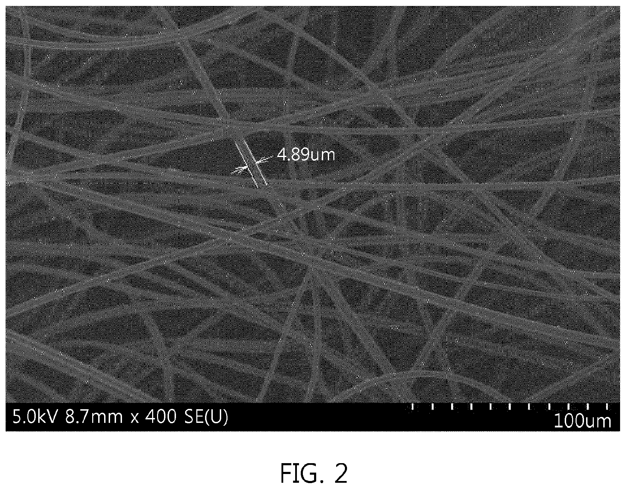

FIG. 2 shows the SEM image of the superabsorbent polymer fiber according to an embodiment of the present invention, as observed using a table SEM, for example, a Phenom Pro model available from PHENOM WORLD. Based on the results of magnified observation of the superabsorbent polymer fiber, a single strand of the fiber was measured to have a width of 4.89 µm.

As is apparent from the above results, the superabsorbent polymer fiber according to the present invention can be found to be a novel type of fiber having a superabsorbent polymer function in various CRC and AUL distribution ranges.

## Claims

1. A method of manufacturing a superabsorbent polymer fiber, comprising steps of:
1) preparing a neutralization solution by dissolving a polymer of a water-soluble ethylenic unsaturated monomer in a sodium hydroxide aqueous solution;
2) preparing a spinning solution by adding the neutralization solution with a crosslinking agent and then performing stirring; and
3) producing a superabsorbent polymer fiber by subjecting the spinning solution to centrifugal spinning using a spinneret and then performing drying,
wherein the neutralization solution has a neutralization degree of 40 to 90 mol%,
wherein the water-soluble ethylenic unsaturated monomer is at least one selected from the group consisting of isobutylene, maleic anhydride, acrylic acid, methylacrylate, and hydroxypropyl methacrylate, and
wherein the crosslinking agent is an epoxy compound.

2. The method of claim 1, wherein the neutralization solution has a neutralization degree of 50 to 80 mol%.

3. The method of claim 1, wherein the centrifugal spinning is performed at a rotation speed of 3,000 rpm to 15,000 rpm.

4. The method of claim 1, wherein in the preparing the neutralization solution of step 1), the water-soluble ethylenic unsaturated monomer is used in an amount of 10 to 50 wt% based on a total weight of the sodium hydroxide aqueous solution.

5. The method of claim 1, wherein in the preparing the spinning solution of step 2), the crosslinking agent is used in an amount of 0.001 to 5 wt% based on a total weight of the water-soluble ethylenic unsaturated monomer.

6. The method of claim 1, wherein in step 3), the drying is performed at a temperature of 100 to 250°C for 10 min to 120 min.

## Patentansprüche

1. Verfahren zum Herstellen einer Superabsorberpolymerfaser, umfassend die Schritte:
1) Herstellen einer Neutralisierungslösung durch Auflösen eines Polymers eines wasserlöslichen ethylenischen ungesättigten Monomers in einer wässrigen Natriumhydroxidlösung;
2) Herstellen einer Spinnlösung durch Zufügen zu der Neutralisierungslösung eines Vernetzungsmittels und dann Durchführen eines Rührens; und
3) Herstellen einer Superabsorberpolymerfaser durch Unterziehen der Spinnlösung einem Zentrifugalspinnen unter Verwendung einer Spinndüse und dann Durchführen eines Trocknens,
wobei die Neutralisierungslösung einen Neutralisationsgrad von 40 bis 90 mol% aufweist,
wobei das wasserlösliche ethylenische ungesättigte Monomer wenigstens eines ist, ausgewählt aus der Gruppe bestehend aus Isobutylen, Maleinsäureanhydrid, Acrylsäure, Methacrylat und Hydroxypropylmethacrylat, und
wobei das Vernetzungsmittel eine Epoxyverbindung ist.

2. Verfahren nach Anspruch 1, wobei die Neutralisierungslösung einen Neutralisationsgrad von 50 bis 80 mol% aufweist.

3. Verfahren nach Anspruch 1, wobei das Zentrifugalspinnen bei einer Drehgeschwindigkeit von 3.000 Upm bis 15.000 Upm durchgeführt wird.

4. Verfahren nach Anspruch 1, wobei beim Herstellen der Neutralisierungslösung von Schritt 1) das wasserlösliche ethylenische ungesättigte Monomer in einer Menge von 10 bis 50 Gew.-%, basierend auf einem Gesamtgewicht der wässrigen Natriumhydroxidlösung, verwendet wird.

5. Verfahren nach Anspruch 1, wobei beim Herstellen der Spinnlösung von Schritt 2) das Vernetzungsmittel in einer Menge von 0,001 bis 5 Gew.-%, basierend auf einem Gesamtgewicht des wasserlöslichen ethylenischen ungesättigten Monomers, verwendet wird.

6. Verfahren nach Anspruch 1, wobei in Schritt 3) das Trocknen bei einer Temperatur von 100 bis 250°C für 10 min bis 120 min durchgeführt wird.

## Revendications

1. Procédé de fabrication d'une fibre de polymère superabsorbant, comprenant les étapes consistant à :
1) préparer une solution de neutralisation en dissolvant un polymère d'un monomère insaturé éthylénique soluble dans l'eau dans une solution aqueuse d'hydroxyde de sodium ;
2) préparer une solution de filage en ajoutant à la solution de neutralisation un agent de réticulation, puis en effectuant une agitation ; et
3) produire une fibre de polymère superabsorbant en soumettant la solution de filage à un filage centrifuge utilisant une filière, puis en effectuant un séchage,
dans lequel la solution de neutralisation a un degré de neutralisation compris entre 40 et 90 % en moles,
dans lequel le monomère insaturé éthylénique soluble dans l'eau est un ou plusieurs éléments sélectionnés dans un groupe constitué de l'isobutylène, l'anhydride maléique, l'acide acrylique, le méthylacrylate et le méthacrylate d'hydroxypropyle, et
dans lequel l'agent de réticulation est un composé époxy.

2. Procédé selon la revendication 1, dans lequel la solution de neutralisation a un degré de neutralisation compris entre 50 et 80 % en moles.

3. Procédé selon la revendication 1, dans lequel le filage centrifuge est effectué à une vitesse de rotation comprise entre 3 000 tr/mn et 15 000 tr/mn.

4. Procédé selon la revendication 1, dans lequel dans la préparation de la solution de neutralisation de l'étape 1), le monomère insaturé éthylénique soluble dans l'eau est utilisé dans une quantité comprise entre 10 et 50 % en poids sur la base d'un poids total d'une solution aqueuse d'hydroxyde de sodium.

5. Procédé selon la revendication 1, dans lequel dans la préparation de la solution de filage de l'étape 2), l'agent de réticulation est utilisé dans une quantité comprise entre 0,001 et 5 % en poids sur la base d'un poids total du monomère insaturé éthylénique soluble dans l'eau.

6. Procédé selon la revendication 1, dans lequel à l'étape 3), le séchage est effectué à une température comprise entre 100 et 250°C pendant 10 min à 120 min.
